# EUROPÄISCHE PATENTSCHRIFT

(11) **EP 1 945 280 B1**
(45) Veröffentlichungstag und Bekanntmachung des Hinweises auf die Patenterteilung: **16.12.2009**
(21) Anmeldenummer: 06806408.8
(22) Anmeldetag: 19.10.2006
(51) Int. Cl.: A61L 31/08

(54) **IMPLANTAT, INSBESONDERE STENT, UND VERFAHREN ZUM HERSTELLEN EINES SOLCHEN IMPLANTATS**
IMPLANT, PARTICULARLY STENT, AND METHOD FOR THE PRODUCTION OF SUCH AN IMPLANT
IMPLANT, EN PARTICULIER STENT, ET PROCEDE POUR REALISER CET IMPLANT

(30) Priorität: 08.11.2005 DE 102005053247
(43) Veröffentlichungstag der Anmeldung: 23.07.2008
(73) Patentinhaber: Fricke, Martin, 99091 Erfurt (DE)
(72) Erfinder: Fricke, Martin, 99091 Erfurt (DE)
(74) Vertreter: Volpert, Marcus
(86) Internationale Anmeldenummer: PCT/EP2006/010105
(87) Internationale Veröffentlichungsnummer: WO 2007/054192

(56) Entgegenhaltungen:
- EP-A- 1 614 442
- EP-A- 1 652 963
- WO-A-2006/043166
- JP-A- 2002 102 330
- JP-A- 2005 095 584
- HAN Y, XU K: "Photoexcited formation of bone apatite-like coatings on micro-arc oxidized titanium" JOURNAL OF BIOMEDICAL MATERIALS RESEARCH. PART A., Bd. 71, Nr. 4, 15. Dezember 2004 (2004-12-15), Seiten 608-614, XP002463533
- TANG G-X ET AL: "Preparation of porous anatase titania film" MATERIALS LETTERS, NORTH HOLLAND PUBLISHING COMPANY. AMSTERDAM, NL, Bd. 58, Nr. 12-13, Mai 2004 (2004-05), Seiten 1857-1860, XP004498234 ISSN: 0167-577X

## Beschreibung

Die Erfindung bezieht sich auf ein Implantat, insbesondere Stent, dessen Oberfläche mit einer Dünnschicht beschichtet ist, sowie auf ein Verfahren zum Herstellen eines solchen Implantats, insbesondere eines Stents, nach dem Oberbegriff des Patentanspruchs 10.

Aus der EP 1 535 660 A1 ist eine Vorrichtung zum Erzeugen wenigstens eines fluiden Reaktionsproduktes aus wenigstens einem fluiden Ausgangsstoff mittels chemischer Reaktion im Plasma dielektrisch behinderter Entladungen bekannt. Diese Vorrichtung hat eine erste Elektrode aus einem porösen, elektrisch leitfähigen Körper, eine zweite Elektrode aus einem ebensolchen Körper und eine zwischen den durchströmbaren Elektroden vorgesehene dielektrische Schicht. Die dielektrische Schicht ist eine Dünnschicht, vorzugsweise eine photokatalytische Dünnschicht aus Titandioxid (TiO₂), besonders bevorzugt aus dem TiO₂-Mineral Anatas. Jede der genannten Elektroden weist zwischen ihrem porösen Körper und der dielektrischen Schicht dieses Körpers eine vorzugsweise aus Titan (Ti) als Haftvermittler gebildete Übergangsschicht auf. Diese Vorrichtung eignet sich gemäß dem vorgenannten Dokument dazu, im Falle von mit Brennstoffzellen betriebenen Kraftfahrzeugen große Mengen an Wasserstoff aus Kohlenwasserstoffen, insbesondere aus Methan oder Erdgas, Flüssiggasen, vergastem Benzin oder vergastem Diesel, herzustellen, wobei ein solcher Prozess on-board, d.h. unmittelbar im Kraftfahrzeug, erfolgen kann. Die hier beschriebene Vorrichtung eignet sich also zur Herstellung insbesondere von wasserstoffreichem Brenngas für mit Brennstoffzellen ausgerüsteten Kraftfahrzeugen.

Aus der DE 102 10 465 A1 ist ein photokatalytisches Element zur Aufspaltung von Wasserstoff enthaltenden Verbindungen und ein Verfahren zur Herstellung eines solchen photokatalytischen Elementes bekannt. Bei dem photokatalytischen Element ist eine photokatalytisch wirkende bindemittelfreie Dünnschicht aus einem photohalbleitenden Material auf einem Träger ausgebildet, und der Träger weist eine offenporige Struktur auf oder bildet eine solche offenporige Struktur. Bei dem letztgenannten Verfahren wird auf einem eine offenporige Struktur aufweisenden Träger eine photokatalytisch wirkende bindemittelfreie Dünnschicht mittels eines plasmagestützten Vakuumbeschichtungsverfahrens ausgebildet. Auch in diesem Dokument ist besonderer Bezug genommen auf den Betrieb von Brennstoffzellen. Es ist ferner erwähnt, die genannten photokatalytischen Elemente zur Deodorierung oder Desinfektion beispielsweise von Abluft aus industriellen oder landwirtschaftlichen Prozessen zu nutzen oder zur Reinigung von mit halogenorganischen Verbindungen kontaminiertem Wasser einzusetzen oder die kanzerogenen oder mutagenen Wirkungen derartiger Verbindungen zu beseitigen. Ferner wird gemäß dieser Schrift die photokatalytische Dünnschicht aus Titandioxid der Anatase-Modifikation durch ein reaktives Pulsmagnetronsputtern ausgebildet. Dabei kann eine Zwischenschicht aus hochreinem Titan bestehen, welche beispielsweise mittels eines Sputterprozesses gebildet wird.

Aus der DE 601 06 962 T2 ist ein poröser, metallischer Stent bekannt, der mit einer keramischen Schicht beschichtet ist und einen pharmakologisch aktiven Wirkstoff aufweist, wobei die Poren des Stents die Fähigkeit haben, pharmakologisch aktive Wirkstoffe aufzunehmen und zu eluieren. Die keramische Schicht kann aus Titandioxid (TiO₂) bestehen. Dieses Dokument offenbart ein Verfahren für die Erzeugung einer Polymerbeschichtung oder keramischen Beschichtung des porösen metallischen Stents mit Hilfe von Verfahren der punktuellen Filmablagerung (spotted film deposition) und damit eine deutliche Modifikation der Oberfläche eines metallischen Stents, um die fortgesetzte Abgabe von Medikamenten in unterschiedlicher Intensität aus dem Stent zu ermöglichen. JP 2002 102 330, JP 2005 095584 und WO 2005/043166 offenbaren Implantate die beschichtet sind mit Oxiden von Titan und Anatas, und Verfahren zum Herstellen dieser Implantate.

Ferner ist aus der DE 102 43 132 A1 ein Verfahren zur Herstellung einer biokompatiblen metallionenhaltigen Titanoxid-Beschichtung auf einem Implantat bekannt, wobei die Metallionen unter physiologischen Bedingungen eluierbar und homogen in der Beschichtung verteilt sind. Ferner ist aus dieser Schrift ein Verfahren zum Herstellen eines solchen Implantats bekannt. Unter Titanoxid wird hier im Wesentlichen Titandioxid verstanden. Durch das hier beschriebene Verfahren wird eine Titanoxid-Beschichtung bzw. ein Implantat mit einer Titanoxid-Beschichtung geschaffen, wobei die Metallionen mit antimikrobieller Wirkung unter physiologischen Bedingungen herauslösbar sind. Nach einer gewissen Zeit, wenn die antimikrobiell wirkenden Metallionen im Wesentlichen herausgelöst sind, nimmt die antimikrobielle Wirkung der Beschichtung ab, und das Implantat wird vom Körpergewebe integriert, ist also biokompatibel.

Die sogenannte perkutane transluminale Angioplastie (PTA) von Blutgefäßen, insbesondere von koronaren Arterien, dient dazu, Verengungen oder sogenannte Stenosen zu beseitigen, welche die Blutversorgung beispielsweise menschlicher Organe behindern. Eine übermäßige Neubildung der inneren Gefäßwand, Gefäßintima genannt, innerhalb des Stents gilt als Hauptursache für eine Restenose, also für ein erneutes Verengen des betreffenden Blutgefäßes.

Der Erfindung liegt die Aufgabe zugrunde, ein Implantat, insbesondere einen Stent, zu schaffen, dessen Verträglichkeit mit Körpergewebe auf besonders einfache Weise verbessert und dadurch eine Restenose verhindert ist. Ferner liegt der Erfindung die Aufgabe zugrunde, ein Verfahren zum Herstellen eines solchen Implantats, insbesondere eines Stents, anzugeben.

Diese Aufgabe wird durch ein Implantat mit den Merkmalen des Patentanspruchs 1 und durch ein Verfahren mit den Merkmalen des Patentanspruchs 8 gelöst. Vorteilhafte Weiterbildungen sind Gegenstand der jeweiligen Unteransprüche.

Erfindungsgemäß hat das Implantat, insbesondere der Stent, eine Oberfläche, die mit einer Dünnschicht aus Titan (Ti) und Titandioxid (TiO₂) beschichtet ist, wobei die Dünnschicht aus einer auf die Oberfläche des Basismaterials des Implantats aufgebrachten Zwischenschicht aus reinem Titan (Ti) als Verbindung zwischen dem Basismaterial des Implantats und der Oberflächenschicht und einer äußeren Oberflächenschicht aus dem TiO₂-Mineral Anatas besteht. Die Oberflächenschicht ist durch Photoanregung photokatalytisch ausgebildet. Ein Implantat mit einer derartigen Dünnschicht weist eine gute Bioverträglichkeit und eine ebenso gute Langzeitverträglichkeit im Zusammenhang mit Körpergewebe auf. Neben verbesserten Gleiteigenschaften und einer ausgezeichneten, sicheren Haftung der Dünnschicht auf metallischen und auch auf nichtmetallischen Basismaterialien des Implantats, auch Trägermaterialien genannt, weist das erfindungsgemäße Implantat eine gute Langzeitakzeptanz und ein gutes Einwachsen im Gewebe, bei Stents in der Gefäßwand, auf. Erfindungsgemäß wird also die verbesserte Verträglichkeit des Implantats nicht wie im vorgenannten Stand der Technik durch pharmakologisch aktive Wirkstoffe oder durch herauslösbare Metallionen mit antimikrobieller Wirkung sondern allein durch den physikalischen Aufbau der Dünnschicht und die sich daraus ergebenden Vorteile erreicht.

Durch die Photoanregung der äußeren Oberflächenschicht wird diese superhydrophil, wodurch die Gleiteigenschaften verbessert und Anlagerungen, wie z.B. bei einer Thrombose, verhindert werden. Ferner werden durch die Photoanregung der äußeren Oberfläche über photokatalytische Prozesse an der Grenzfläche der photosensiblen Oberflächenschicht und dem Intimagewebe des Gefäßes Substanzen erzeugt, welche die Restenose verhindern oder zurückbilden. Die sogenannte photoaktivierbare Superhydrophilie verbessert ebenfalls die Gleiteigenschaften bei der Angioplastie und verringert das akute Thromboserisiko. Aufgrund der Superhydrophilie werden auch Anlagerungen an dem Implantat-Basismaterial vermieden.

Die Zwischenschicht aus reinem Titan (Ti) ist eine Art Verbindungsschicht, welche die äußere Oberflächenschicht aus dem TiO₂-Mineral Anatas fest mit dem Basismaterial des Implantats verbindet, so dass die äußere Oberflächenschicht eine fixierte oder immobilisierte Oberflächenschicht darstellt. Diese Zwischenschicht aus reinem Titan ist aber nicht nur Verbindungs- und Zwischenschicht sondern wirkt auch als sogenannte Barriereschicht für die Metallionen (sofern vorhanden) des Basismaterials des Implantats. Diese können also nicht nach außen und daher nicht, wie bei dem vorerwähnten Stand der Technik erwähnt, in das Körpergewebe bzw. in die Blutbahn gelangen. Die Zwischenschicht aus Titan weist duktile Eigenschaften auf, wohingegen die äußere Oberflächenschicht aus dem erwähnten TiO₂-Mineral keramisch aufgebaut ist und eine monokristalline Struktur hat.

Gemäß einer anderen bevorzugten Weiterbildung der Erfindung sind die Kanten des Basismaterials des Implantats abgerundet. Dadurch wird die Gefäßinnenwand beim Einsetzen des Stents möglichst wenig gereizt, wodurch das Restenoserisiko geringer gehalten werden kann. Ein erneutes Verengen bzw. Verschließen des Gefäßes ist dadurch weitgehend minimiert. Ein derart ausgestaltetes, erfindungsgemäßes Implantat kann daher besonders schonend an die gewünschte Stelle beispielsweise in einem Blutgefäß eingesetzt werden. Aufgrund der abgerundeten Kanten des Basismaterials werden außerdem die Gleiteigenschaften des Implantats beispielsweise im Blutgefäß verbessert und die Gefahr von Läsionen der Gefäßwand bei einer PTA verringert.

Vorteilhafterweise beträgt die Dicke der Zwischenschicht 200 bis 1000 nm und vorzugsweise diejenige der äußeren Oberflächenschicht 100 bis 1000 nm. Insofern können die einzelnen Schichten und damit die Dünnschicht insgesamt äußerst dünn gehalten sein.

Gemäß einer anderen Weiterbildung der Erfindung sind die Zwischenschicht und die äußere Oberflächenschicht jeweils eine allseitige Plasmabeschichtung des Implantats. Daraus folgt, dass das Basismaterial des Implantats, insbesondere des Stents, insgesamt also beispielsweise auch an den innen liegenden Oberflächen des Implantats, oberflächenbeschichtet ist/sind. Dadurch hat das erfindungsgemäße Implantat insgesamt und nicht nur an einzelnen Stellen eine gute Bioverträglichkeit, wodurch das Restenoserisiko deutlich reduziert werden kann.

Gemäß einer anderen Weiterbildung der Erfindung ist die Dünnschicht als geschlossene Oberflächenschicht auf der gesamten Oberfläche des deflatierten Implantats, vorzugsweise des Stents, aufgebracht. Daraus folgt, dass sich das Implantat bei der Aufbringung der Dünnschicht vorzugsweise im nicht gespreizten oder im nicht entfalteten Zustand befindet.

Gemäß einer ferner bevorzugten Weiterbildung der Erfindung ist die äußere Oberflächenschicht vorzugsweise durch Beleuchten mit blauem Licht oder UVA-Licht im Wellenlängenbereich zwischen 360 und 460 nm photodynamisch aktivierbar bzw. reaktivierbar, wobei eine Aktivierung bzw. eine In-vivo-Reaktivierung der äußeren Oberflächenschicht mittels einer Lichtleitfaser erfolgen kann, die vorzugsweise eine das Licht in radialer Richtung abstrahlende Faseroptik aufweist. Insofern kann die Photoaktivierung der äußeren Oberflächenschicht unmittelbar vor der Angioplastie durch Photoaktivierung der Hydrophilie mit Hilfe einer einfachen Beleuchtung bzw. Photoanregung der gesamten Implantatoberfläche erfolgen. Es ist ferner möglich, die Hydrophilie bzw. die besonderen Schichteigenschaften zur Nachbehandlung zu einem späteren Zeitpunkt wieder zu reaktivieren, wobei eine derartige Reaktivierung die genannte In-vivo-Reaktivierung sein kann. Dabei können zur Durchführung der photochemischen Prozesse an den Grenzflächen der Innenseite des Implantats, insbesondere des Stents, mit Hilfe von Kathetern in das Implantat, vor allem dem Stent, geschobene Lichtleitfaserkabel und Mikrofaseroptiken zum Einsatz kommen. Insofern kann mit dieser Weiterbildung das Restenoserisiko durch Verhindern der Einwachsprozesse mit Hilfe der genannten Photokatalyse im Rahmen einer In-vivo-Reaktivierung herabgesetzt sein. Die genannte Lichtleitfaser mit ihrer Faseroptik ermöglicht daher ein Bestrahlen des Implantats an dessen Einsatzort, d.h. beispielsweise in einem Blutgefäß wie einem Herzkranzgefäß.

Das erfindungsgemäße Verfahren zum Herstellen eines Implantats, insbesondere eines Stents, weist folgende Vakuumprozessschritte auf: Plasmavorbehandlung mit Abrundung der Kanten des Implantat-Basismaterials; Sputtern einer Zwischenschicht aus Titan (Ti); Sputtern einer Oberflächenschicht aus dem Titandioxid(TiO₂)-Mineral Anatas. Damit kann das Abrunden der Kanten des Implantat-Basismaterials zeitlich vor dem Aufbringen der Dünnschicht auf das Basismaterial erfolgen, so dass das Implantat ausschließlich in abgerundeten Kanten mit der erfindungsgemäßen Dünnschicht versehen wird.

Vorteilhafterweise umfasst die Plasmavorbehandlung eine Plasmaoberflächenreinigung und eine Plasmapolitur. Damit wird letztlich ausschließlich ein hochreines, gut bioverträgliches Implantat mit der speziellen Dünnschicht bestehend aus zwei Oberflächenschichten versehen.

Gemäß einer anderen Weiterbildung der Erfindung werden die Zwischenschicht aus Titan und die Oberflächenschicht aus dem TiO₂-Mineral Anatas durch einen gepulsten, reaktiven Magnetronsputterprozess (reaktive Pulse Magnetron Sputtering, PMS) aufgebracht. Dieses Aufbringen der beiden Schichten erfolgt also auf die gesamte Aussenfläche des mechanisch fertig bearbeiteten Implantats, wie einen Rohstent. Der Sputterprozess umfasst somit auch die innen liegenden Aussenflächen des Implantats.

Ausführungsbeispiele des Erfindungsgegenstandes werden nachfolgend anhand der Zeichnung näher erläutert, wobei alle beschriebenen und/oder bildlich dargestellten Merkmale für sich oder in beliebiger Kombination den Gegenstand der vorliegenden Erfindung unabhängig von ihrer Zusammenfassung in den Ansprüchen oder deren Rückbeziehung bilden. Es zeigen:
- Fig. 1: einen schematischen Längsschnitt durch ein verengtes Gefäß, zum Beispiel ein Herzkranzgefäß;
- Fig. 2: einen schematischen, teilweisen Längsschnitt durch ein verengtes Gefäß mit eingeführtem Ballonkatheter und montiertem Implantat in Form eines Stents;
- Fig. 3: einen schematischen, teilweisen Längsschnitt durch ein ballondilatiertes Gefäß nach einer Expansion des Implantats und entferntem Ballonkatheter;
- Fig. 4: einen schematischen Querschnitt durch einen Steg des Implantats;
- Fig. 5: einen schematischen Querschnitt durch den Steg nach einer Abrundung der Kanten;
- Fig. 6: einen schematischen Querschnitt durch den Steg nach Aufbringen einer Zwischenschicht aus Titan auf den Steg gemäß Fig. 5;
- Fig. 7: einen schematischen Querschnitt durch den Steg nach Aufbringen einer Oberflächenschicht aus dem Titandioxid-Mineral Anatas auf den Steg gemäß Fig. 6;
- Fig. 8: eine beispielhafte, schematische Darstellung eines Implantats im deflatierten Zustand;
- Fig. 9: eine beispielhafte, schematische Darstellung eines Implantats im deflatierten, gebogenen Zustands; und
- Fig. 10: eine beispielhafte, schematische Darstellung eines Implantats im inflatierten, d.h. gespreizten bzw. entfalteten Zustand.

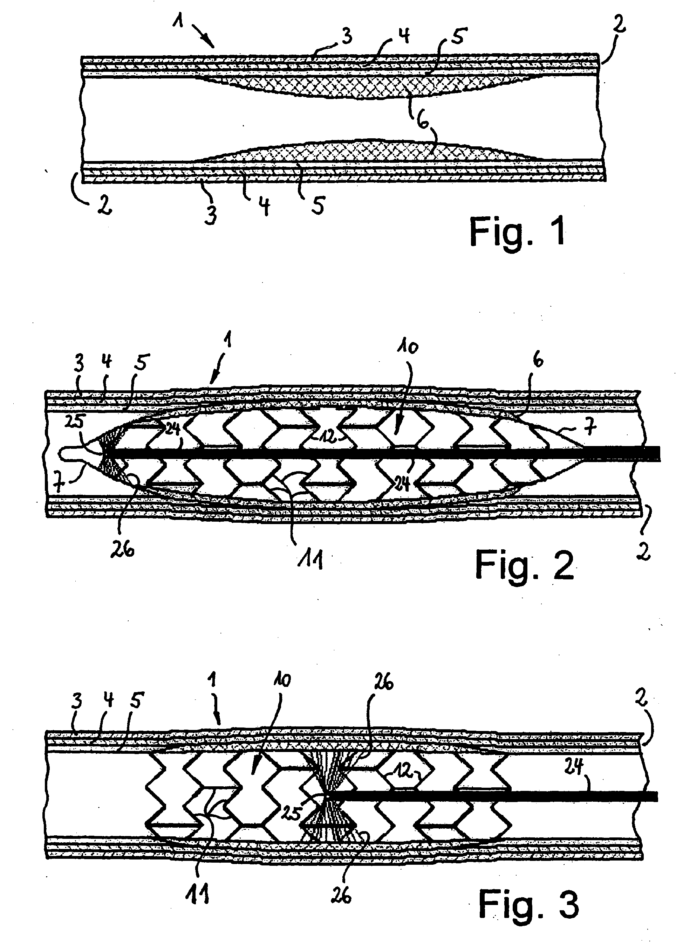

In Fig. 1 ist ein schematischer Längsschnitt durch einen Teil eines Gefäßes 1, z.B. eines Herzkranzgefäßes, gezeigt. Das Gefäß 1 hat eine Gefäßwand 2, welche aus drei Schichten aufgebaut ist. Von radial außen nach radial innen sind dies die äußere Gefäßwandschicht 3, Tunica externa genannt, die mittlere Gefäßwandschicht 4, Tunica media genannt, und die innere Gefäßwandschicht 5, Tunica intima genannt.

Das Gefäß 1 zeigt eine Stenose 6 durch Plaque. Es ist klar, dass der zuvor beschriebene Aufbau des Gefäßes 1 lediglich eine beispielhafte Erläuterung eines solchen Gefäßes darstellt. Ebenso gut können Gefäße mit anderem Aufbau mit umfasst sein.

In Fig. 2 ist ein schematischer Längsschnitt durch das verengte Gefäß 1 mit eingeführtem Ballonkatheter 7 und montiertem Implantat 10, nämlich einem Stent 11, dargestellt.

Das Implantat ist lediglich beispielhaft als Stent ausgebildet. Insofern ist die Erfindung nicht auf ein als Stent ausgebildetes Implantat beschränkt; vielmehr umfasst die Erfindung auch andere Arten von Implantaten.

Die Erfindung umfasst ferner auch medizinische, insbesondere zahnmedizinische Instrumente, deren Oberflächen zumindest teilweise in der erfindungsgemässen Weise aufgebaut sind. Insofern bezieht sich die Erfindung allgemein auf Implantate sowie medizinische Instrumente.

In Fig. 2 ist der Stent 11 teilweise inflatiert, d.h. entfaltet, dargestellt.

Demgegenüber zeigt Fig. 3 einen schematischen, teilweisen Längsschnitt durch das Gefäß 1 mit nunmehr vollständig inflatiertem Stent und entferntem Ballonkatheter 7.

### Auf die Fig. 2 und 3 wird später noch genauer eingegangen:

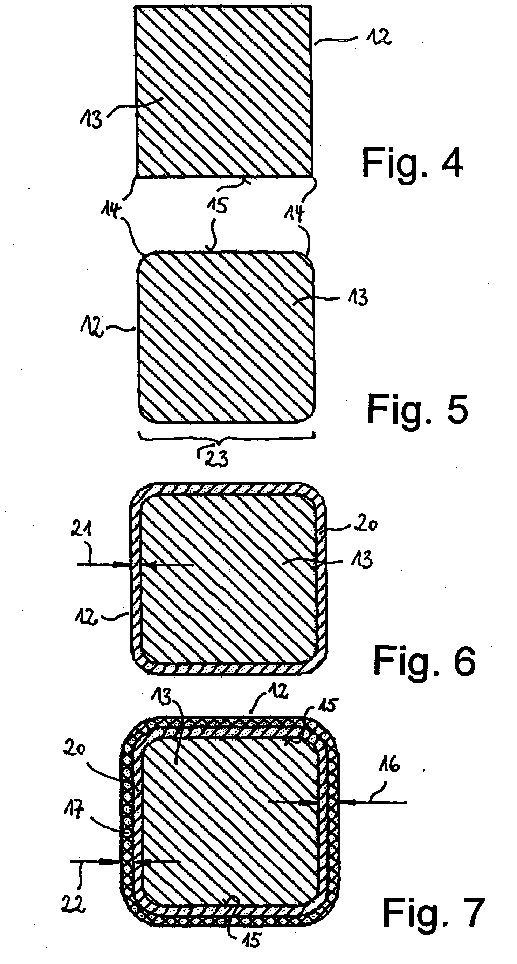

Nachfolgend wird das erfindungsgemäße Implantat 10 mit Bezug auf die Fig. 4 bis 7 genauer erläutert.

Wie zuvor erwähnt ist das Implantat 10 vorzugsweise aber nicht ausschließlich als Stent 11 ausgebildet. Ein solcher Stent 11 hat zahlreiche Stege 12, auch Stützstege (Struts) genannt, von denen einer jeweils im Querschnitt in den Fig. 4 bis 7 dargestellt ist. In Fig. 4 ist ein Querschnitt durch einen solchen Steg des Stents 11 schematisch dargestellt. Der Steg ist beispielsweise aus chirurgischem Stahl, beispielsweise chirurgischem Stahl 316L, oder aus Nitinol^{®}, einer korrosions- und erschütterungsresistenten, stahlharten Titan-Nickel-Legierung, gefertigt. Das Basismaterial 13 des Stents 11 kann aber auch aus anderen Metallen, Legierungen, auch aus Nichtmetallen und Kunststoffen bestehen. Der in Fig. 4 gezeigte Querschnitt durch einen Steg 12 ergibt sich beispielsweise nach Laserausschnitt des Materials aus einem Rohrmantel.

In der Darstellung gemäß Fig. 5 sind die Kanten 14 des Basismaterials 13 des Stegs 12 des Stents 11 abgerundet, worauf später noch näher eingegangen wird.

Erfindungsgemäß hat das Implantat 10, also beispielsweise der Stent 11, eine Oberfläche 15, die mit einer Dünnschicht 16 aus Titan (Ti) und Titandioxid (TiO₂) beschichtet ist, wobei die Dünnschicht 16 aus einer auf die Oberfläche 15 des Basismaterials 13 des Implantats 10 aufgebrachten Zwischenschicht 20 aus Titan (Ti) als Verbindung zwischen dem Basismaterial 13 des Implantats 10 und der Oberflächenschicht 17 und einer äußeren Oberflächenschicht 17 aus den Ti-O₂-Mineral Anatas besteht. Diese Oberflächenschicht 17 ist durch Photoanregung photokatalytisch wirkend ausgebildet.

Die Erfindung betrifft insbesondere eine photoaktivierbare Dünnschichtoberfläche aus dem TiO₂-Mineral Anatas in Form einer monokristallinen Schicht als bioaktive Oberfläche eines Implantats und/oder eines medizinischen Instruments.

Wie zuvor erwähnt, werden die zwei erläuterten Schichten 17, 20 der Dünnschicht 16 auf das abgerundete Basismaterial 13 des Implantats 10 aufgebracht. Daraus folgt, dass vor dem Aufbringen der Dünnschicht 16 zunächst die Kanten 14 bzw. die Ecken des Basismaterials in einem vorgelagerten Bearbeitungsschritt abgerundet werden.

Es wird darauf hingewiesen, dass die Zwischenschicht aus Titan entfallen kann, sofern das Basismaterial des Implantats bzw. des medizinischen Instruments aus Titan gefertigt ist oder zumindest in nennenswertem Umfang Titan enthält.

Die Dicke 21 der Zwischenschicht 20 beträgt 200 bis 1000 nm, und vorzugsweise beträgt die Dicke 22 der äußeren Oberflächenschicht 17 100 bis 1000 nm.

Wie in den Fig. 4 bis 7 lediglich schematisch angedeutet, sind die Zwischenschicht und die äußere Oberflächenschicht 20, 17 jeweils eine allseitige Beschichtung, vorzugsweise eine allseitige Plasmabeschichtung des Implantats 10. Gemäß einer bevorzugten Ausführungsform der Erfindung ist die Dünnschicht 16 als geschlossene Schicht 17, 20 auf der gesamten Oberfläche 15 des deflatierten Implantats 10, vorzugsweise des Stents 11, aufgebracht.

Die Kantenlänge 23 des Basismaterials 13 beträgt vorzugsweise 0,1 mm. Es ist klar, dass die Oberfläche 15, anders als in den Fig. 4 bis 7 gezeigt, auch gekrümmt ausgebildet sein kann.

Gemäß einer besonders bevorzugten Ausführungsform der Erfindung ist die äußere Oberflächenschicht 17 vorzugsweise durch Beleuchten mit blauem Licht oder UVA-Licht im Wellenlängenbereich zwischen 360 und 460 nm photodynamisch aktivierbar bzw. reaktivierbar. Eine solche Aktivierung bzw. Reaktivierung der äußeren Oberflächenschicht 17 kann gemäß der in den Fig. 2 und 3 gezeigten Ausführungsform der Erfindung mittels einer Lichtleitfaser 24 erfolgen. Die Lichtleitfaser 24 hat eine Licht 26 in radialer Richtung abstrahlende Faseroptik 25. Wie in den Fig. 2 und 3 angedeutet, ist die Faseroptik 25 derart ausgebildet, dass das Licht 26 die Lichtleitfaser 24 etwa in radialer Richtung verlässt. Wie ferner in den Fig. 2 und 3 gezeigt, kann die Reaktivierung der äußeren Oberflächenschicht 17 vorzugsweise eine In-vivo-Reaktivierung sein.

Nachfolgend wird das erfindungsgemäße Verfahren zum Herstellen eines Implantats, insbesondere eines Stents, näher erläutert.

Das erfindungsgemäße Verfahren weist folgende Vakuumprozessschritte auf:
- Plamavorbehandlung mit Abrundung der Kanten des Implantat-Basismaterials;
- Sputtern einer Zwischenschicht aus reinem Titan (Ti);
- Sputtern einer Oberflächenschicht aus dem Titandioxid(TiO₂)-Mineral Anatas.

Gemäß einer bevorzugten Weiterbildung der Erfindung umfasst die Plasmavorbehandlung ferner eine Plasmaoberflächenreinigung und eine Plasmapolitur.

Insofern stellt der in Fig. 4 gezeigte Querschnitt eines Stegs 12 des Implantats 10 das Basismaterial 13 in Rohform dar, wohingegen der Querschnitt gemäß Fig. 5 bereits die erwähnte Plasmavorbehandlung, insbesondere eine Abrundung der Kanten, Unebenheiten und des Bearbeitungsgrates des Implantat-Basismaterials, erfahren hat.

Die Schichten 20, 17 aus Titan und dem TiO₂-Mineral Anatas werden durch einen gepulsten reaktiven Magnetronsputterprozess (reactive Pulse Magnetron Sputtering, PMS) aufgebracht. Die Plasmavorbehandlung kann auch die Politur bzw. den Ausgleich von Unebenheiten auf der Oberfläche 15 des Basismaterials 13 beinhalten.

Wie zuvor bereits angedeutet, befindet sich der Stent 11 in Fig. 2 in einem teilweise inflatierten, d.h. expandierten Zustand. Gemäß dieser Darstellung ist der Stent 11 hier noch auf dem Ballonkatheter 7 montiert. Die Stenose 6 ist hier schon im Vergleich zur Darstellung gemäß Fig. 1 radial nach außen gedrückt dargestellt, wodurch, wie in den Fig. 2 und 3 angedeutet, sich eine geringe Erweiterung des Gefäßes 1 ergeben kann.

Das Gefäß 1 gemäß Fig. 3 ist ballondilatiert und in einem Zustand nach einer Expansion des Stents 11 und entferntem Ballonkatheter 7 gezeigt. Der Stent 11 ist somit vollständig inflatiert, d.h. entfaltet. Innerhalb des Ballonkatheters 7 befindet sich gemäß Fig. 2 die Lichtleitfaser 24 mit ihrer Faseroptik 25. Mittels Lichtleitfaser und Faseroptik wird die Aktivierung/Reaktivierung, also die Durchführung eines photodynamischen Prozesses, an der photoaktiven äußeren Oberflächenschicht 17 durch Aufbringen von Licht durchgeführt. In Fig. 3 ist die Lichtleitfaser 24 mit ihrer Faseroptik 25 ohne den Ballonkatheter 7 gezeigt. Der Stent ist also in der Lage, die durch eine Stenose 6 herbeigeführte Querschnittsverengung, wie sie in Fig. 1 angedeutet ist, in seinem inflatierten Zustand vollständig zu beseitigen (siehe Darstellung gemäß Fig. 3).

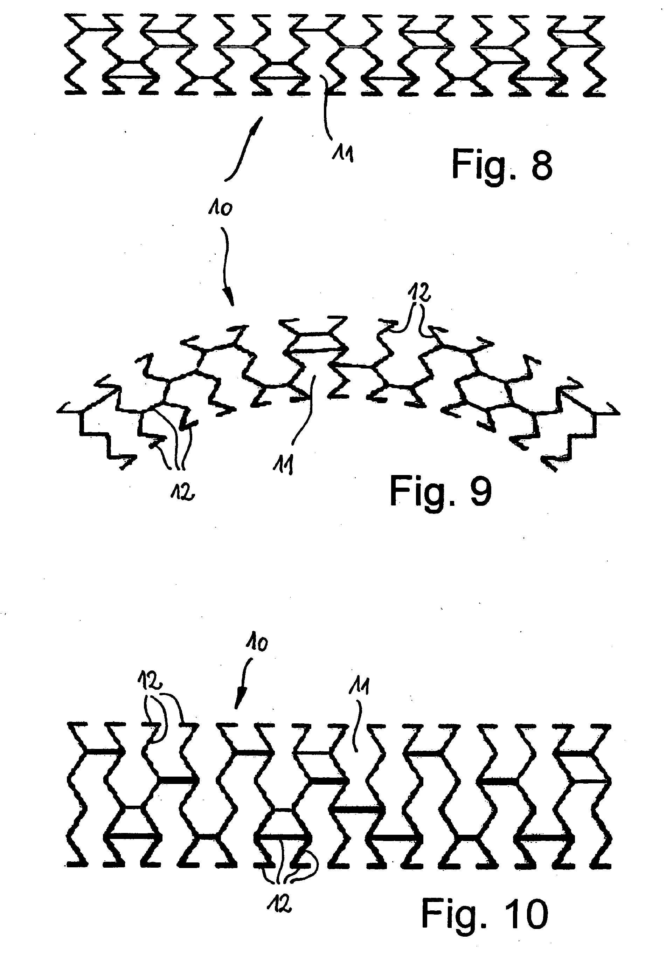

In Fig. 8 ist das Implantat 10 beispielhaft in Form eines Stents 11 in einem deflatierten, d.h. zusammengepressten bzw. -gefalteten Zustand gezeigt. Gemäß Fig. 9 hat ein wie in Fig. 8 deflatierter Stent eine Bogenform, wohingegen in Fig. 10 ein Stent 11 im inflatierten, d.h. gespreizten bzw. entfalteten Zustand gezeigt ist. Etwa in dem in Fig. 10 gezeigten Zustand befindet sich der Stent 11 in dem Gefäß 1 gemäß Fig. 3. Diese Darstellungen verdeutlichen die leichte Beweglichkeit, wie Biegbarkeit, und gute Flexibilität des Implantats.

Es ist klar, dass das Implantat 10 bzw. der Stent 11 zahlreiche unterschiedliche Formen annehmen und insofern in einer Vielzahl von Ausführungsformen gebildet seinkann. Die Stents sind in den Fig. 8 bis 10 lediglich beispielhaft dargestellt.

Insofern kann der Einsatz von Licht zur In-vivo-Aktivierung bzw. -Reaktivierung von Implantatoberflächen einen Beitrag zur minimalinvasiven Medizin darstellen. Wie zuvor erwähnt, ist es möglich, das erfindungsgemäße Implantat mit bereits photokatalytisch angeregter äußerer Oberflächenschicht in das Blutgefäß 1 einzusetzen und den Vorgang der erneuten Aktivierung bzw. Reaktivierung der äußeren Oberflächenschicht im eingebauten Zustand des Stents im Blutgefäß ein weiteres Mal durchzuführen.

Der Stent 11 ist beispielsweise eine implantierbare Gefäßstütze aus Drahtgeflecht oder Metallröhrchen mit Aussparungen in der Rohrwand zur Behandlung von Verschlüssen und Verengungen in Gefäßen. Wie zuvor bereits erwähnt, ist es mittels der Erfindung möglich, die akuten Risiken bei der PTA weiter zu minimieren und insbesondere das Restenoserisiko nach einer PTA zu verringern, wobei dies durch die erwähnte Photoaktivierung bzw. In-vivo-Reaktivierung der photoaktiven Grenzflächen der Oberflächenschicht der Stege erfolgt. Die Reaktivierung kann somit an photoaktivierten Grenzflächen der Oberflächenschicht der Stege mit der Intima und der gegebenenfalls neu gebildeten, wachsenden Neointima durchgeführt werden, um die erwähnte Restenose zu unterbinden oder doch zumindest stark einzuschränken. Die Inflatierung, d.h. die Entfaltung, des Stents kann durch die erwähnte Ballonmontage oder durch Selbstexpansion in vivo erfolgen.

Die Zwischenschicht 20 aus reinem Titan hat die Aufgabe, die Dehnungskoeffizienten des Basismaterials 13, auch Trägermaterial genannt, der Stentstege und der photoaktiven äußeren Oberflächenschicht aus dem genannten TiO₂-Mineral Anatas anzupassen und die letztgenannte Anatas-Schicht fest haftend mit dem Basismaterial zu verbinden. Die Zwischenschicht 20 aus reinem Titan hat ferner die Aufgabe, die bei der plastischen Verformung der Stege des Stents infolge der Inflatation, d.h. Entfaltung, Spreizung, auftretenden Kräfte bzw. Spannungen, wie z.B. Zug-, Druckspannungen, Torsionsspannungen, aufzunehmen, Ablationen zu vermeiden und bei den sich bildenden Mikrorissen in der Schicht Korrosionen zu verhindern sowie das Wachstum (Rekristallisation) von neuen Anatas-Nanokristalliten in den Mikrorissen zur Ausheilung der Schichtoberfläche zu ermöglichen. Das erwähnte TiO₂-Mineral Anatas liegt in einer Schicht aus Monokristalliten der Anatas-Morphologie des TiO₂ als photoaktivierbare Oberfläche vor.

Die genannten Materialien Ti sowie TiO₂ sind hämo- und histokompatible Werkstoffe und als Implantatmaterialien langzeiterprobt.

Insofern kann das erfindungsgemäße Implantat eine Alternative zu medikamentenabgebenden Implantaten, insbesondere Stents, darstellen und in einer einfachen und preiswerten Variante die Möglichkeit bieten, eine Restenose zu vermeiden.

Damit ist ein Implantat bzw. allgemein ein medizinischer Artikel, wie z.B. ein medizinisches Instrument, geschaffen, dessen Verträglichkeit, vor allem Bioverträglichkeit, auf einfache Weise verbessert und das bzw. der in der Lage ist, insbesondere eine Restenose weitgehend zu verhindern. Zusätzlich ist ein Verfahren zum Herstellen eines solchen Implantats bzw. medizinischen Artikels angegeben.

## Patentansprüche

1. Implantat, insbesondere Stent (11),
dessen Oberfläche (15) mit einer Dünnschicht (16) aus Titan (Ti) und Titandioxid (TiO₂) beschichtet ist,
wobei die Dünnschicht (16) aus einer auf die Oberfläche (15) des Basismaterials (13) des Implantats (10) aufgebrachten Zwischenschicht (20) aus Titan (Ti) als Verbindung zwischen dem Basismaterial (13) des Implantats (10) und der Oberflächenschicht (17) und einer äußeren Oberflächenschicht (17) aus dem TiO₂-Mineral Anatas besteht,
**dadurch gekennzeichnet, dass**
die Zwischenschicht (20) aus reinem Titan (Ti) gebildet ist und
die Oberflächenschicht (17) durch Photoanregung photokatalytisch wirkend ausgebildet ist.

2. Implantat nach Anspruch 1, **dadurch gekennzeichnet, dass** die Kanten (14) des Basismaterials (13) des Implantats (10) abgerundet sind.

3. Implantat nach Anspruch 1 oder 2, **dadurch gekennzeichnet, dass** die Dicke (21) der Zwischenschicht (20) 200 bis 1000 nm und vorzugsweise diejenige (22) der Oberflächenschicht (17) 100 bis 1000 nm beträgt.

4. Implantat nach einem der vorhergehenden Ansprüche, **dadurch gekennzeichnet, dass** die Zwischenschicht (20) und die Oberflächenschicht (17) jeweils eine allseitige Plasmabeschichtung des Implantats (10) sind.

5. Implantat nach einem der vorhergehenden Ansprüche, **dadurch gekennzeichnet, dass** die Dünnschicht (16) als geschlossene Schicht (17, 20) auf der gesamten Oberfläche (15) des Implantats (10), vorzugsweise des Stents (11), aufgebracht ist, wobei das Implantat (10) inflatierbar ausgebildet ist und sich beim Aufbringen der Dünnschicht (16) im deflatierten Zustand befindet.

6. Implantat nach einem der vorhergehenden Ansprüche, **dadurch gekennzeichnet, dass** die Oberflächenschicht (17) vorzugsweise durch Beleuchten mit blauem Licht oder UVA-Licht im Wellenlängenbereich zwischen 360 und 460 nm photodynamisch aktivierbar bzw. reaktivierbar ist.

7. Implantat nach Anspruch 6, **dadurch gekennzeichnet, dass** eine Aktivierung bzw. eine Reaktivierung, vorzugsweise In-vivo-Reaktivierung, der Oberflächenschicht (17) mittels einer Lichtleitfaser (24) erfolgt, die vorzugsweise eine das Licht (26) in radialer Richtung abstrahlende Faseroptik (25) aufweist.

8. Verfahren zum Herstellen eines Implantats, insbesondere eines Stents, nach einem der vorhergehenden Ansprüche,
**gekennzeichnet durch** folgende Vakuumprozessschritte:
- Plasmavorbehandlung mit Abrundung der Kanten des Implantat-Basismaterials;
- Sputtern einer Zwischenschicht aus reinem Titan (Ti);
- Sputtern einer Oberflächenschicht aus dem Titandioxid(TiO₂)-Mineral Anatas.

9. Verfahren nach Anspruch 8, **dadurch gekennzeichnet, dass** die Plasmavorbehandlung eine Plasmaoberflächenreinigung und eine Plasmapolitur umfasst.

10. Verfahren nach Anspruch 8 oder 9, **dadurch gekennzeichnet, dass** die Schichten aus Titan und dem TiO₂-Mineral Anatas durch einen gepulsten, reaktiven Magnetronsputterprozess (reaktive Pulse Magnetron Sputtering, PMS) aufgebracht werden.

## Claims

1. Implant, particularly a stent (11), the surface (15) of which is coated with a thin layer (16) made of titanium (Ti) and titanium dioxide (TiO₂), the thin layer (16) consisting of an intermediate layer (20), which is deposited on the surface (15) of the base material (13) of the implant (10) and is made of titanium (Ti) as connection between the base material (13) of the implant (10) and the surface layer (17), and of an outer surface layer (17) made of the TiO₂ mineral anatase, **characterized in that** the intermediate layer (20) is made of pure titanium (Ti) and the surface layer (17) comprises a photocatalytic effect made by photoexcitation.

2. Implant according to claim 1, **characterized in that** the edges (14) of the base material (13) of the implant (10) are rounded.

3. Implant according to claim 1 or 2, **characterized in that** the thickness (21) of the intermediate layer (20) is 200 to 1000 nm and preferably the thickness (22) of the surface layer (17) is 100 to 1000 nm.

4. Implant according to one of the preceding claims, **characterized in that** the intermediate layer (20) and the surface layer (17) are each an all-sided plasma coating of the implant (10).

5. Implant according to one of the preceding claims, **characterized in that** the thin layer (16) is deposited as a closed layer (17, 20) on the entire surface (15) of the implant (10), preferably of the stent (11), the implant (10) made inflatable and being in a deflated state during deposition of the thin layer (16).

6. Implant according to one of the preceding claims, **characterized in that** the surface layer (17) can be photodynamically activated or reactivated preferably by illuminating with blue light or UVA light in the wavelength range between 360 and 460 nm.

7. Implant according to claim 6, **characterized in that** an activation or a reactivation, preferably an in vivo reactivation, of the surface layer (17) takes place by means of an optical fiber (24), which preferably has fiber optics (25) that emit the light (26) in the radial direction.

8. Method for producing an implant, particularly a stent, according to one of the preceding claims, **characterized by** the following vacuum process steps:
- plasma pretreatment with rounding of the edges of the implant base material;
- sputtering of an intermediate layer made of pure titanium (Ti);
- sputtering of a surface layer made of the titanium dioxide (TiO₂) mineral anatase.

9. Method according to claim 8, **characterized in that** the plasma pretreatment comprises a plasma surface treatment and a plasma polishing.

10. Method according to claim 8 or 9, **characterized in that** the layers made of titanium and of the TiO₂ mineral anatase are deposited by reactive Pulse Magnetron Sputtering (PMS).

## Revendications

1. Implant, en particulier stent (11), dont la surface (15) est revêtue d'une couche mince (16) en titane (Ti) et en oxyde de titane (TiO₂), ladite couche mince (16) est composée d'une couche intermédiaire (20) appliquée sur la surface (15) du matériau de base (13) de l'implant (10) en titane (Ti) à titre de liaison entre le matériau de base (13) de l'implant (10) et la couche de surface (17), et d'une couche de surface extérieure (17) réalisée à partir du minéral Anatas à base de TiO₂,
**caractérisé en ce que**
la couche intermédiaire (20) est formée en titane pur (Ti), et
la couche de surface (17) est réalisée par photoexcitation de manière à avoir un effet photocatalytique.

2. Implant selon la revendication 1, **caractérisé en ce que** les arêtes (14) du matériau de base (17) de l'implant (10) sont arrondies.

3. Implant selon la revendication 1 ou 2, **caractérisé en ce que** l'épaisseur (21) de la couche intermédiaire (20) est de 200 à 1000 nm, et **en ce que** de préférence l'épaisseur (22) de la couche de surface (17) est de 100 à 1000 nm.

4. Implant selon l'une des revendications précédentes, **caractérisé en ce que** la couche intermédiaire (20) et la couche de surface (17) sont respectivement un revêtement de l'implant (10) sur tous les côtés au plasma.

5. Implant selon l'une des revendications précédentes, **caractérisé en ce que** la couche mince (16) est appliquée sous forme d'une couche fermée (17, 20) sur la totalité de la surface (15) de l'implant (10), de préférence du stent (11), et l'implant (10) est réalisé gonflable et se trouve dans l'état dégonflé lors de l'application de la couche mince (16).

6. Implant selon l'une des revendications précédentes, **caractérisé en ce que** la couche de surface (17) est susceptible d'être activée ou respectivement réactivée par voie photodynamique, de préférence par illumination à la lumière bleue ou à la lutte lumière ultraviolette dans une plage de longueurs d'onde entre 360 et 460 nm.

7. Implant selon la revendication 6, **caractérisé en ce qu'**une activation ou respectivement une réactivation, de préférence une réactivation in vivo, de la couche de surface (17) a lieu au moyen d'une fibre guide d'onde (24), qui comprend de préférence une optique à fibre (25) qui émet la lumière (26) en direction radiale.

8. Procédé pour fabriquer un implant, en particulier un stent, selon l'une des revendications précédentes,
**caractérisé par** les étapes de traitement suivantes sous vide :
- traitement au plasma avec arrondissement des arêtes du matériau de base de l'implant ;
- pulvérisation d'une couche intermédiaire en titane pur (Ti) ;
- pulvérisation d'une couche de surface du minéral Anatas à base de dioxyde de titane (TiO₂).

9. Procédé selon la revendication 8, **caractérisé en ce que** le traitement au plasma comprend un nettoyage de surface au plasma et un polissage au plasma.

10. Procédé selon la revendication 8 ou 9, **caractérisé en ce que** les couches de titane et de minéral Anatas à base de TiO₂ sont appliquées par un processus de pulvérisation pulsé réactif avec magnétron (reactive Pulse Magnetron Sputtering, PMS).
